# EUROPÄISCHE PATENTANMELDUNG

(11) **EP 3 588 530 A1**
(43) Veröffentlichungstag der Anmeldung: **01.01.2020**
(21) Anmeldenummer: 18180451.9
(22) Anmeldetag: 28.06.2018
(51) Int. Cl.: H01J 5/18, H01J 35/18

(54) **STRAHLENDURCHTRITTSFENSTER UND VERFAHREN ZU DESSEN HERSTELLUNG**

(71) Anmelder: Siemens Healthcare GmbH, 91052 Erlangen (DE)
(72) Erfinder: Engelke, Clemens, 90409 Nürnberg (DE)

(57) **Zusammenfassung**

Die Erfindung betrifft ein Strahlendurchtrittsfenster (1), das aus einem Schichtverbund-Werkstoff (2) besteht, der ein erstes Material (21) und ein zweites Material (22) umfasst, wobei das erste Material (21) ein Trägermaterial ist, das einen Fensterrahmen (3) bildet, und das zweite Material (22) ein strahlendurchlässiges Material ist. Die Erfindung betrifft weiterhin ein Herstellungsverfahren für ein derartiges Strahlendurchtrittsfenster (1). Das erfindungsgemäße Strahlendurchtrittsfenster (1) ist auf einfache Weise auf den jeweiligen Anwendungsfall anpassbar.

## Beschreibung

Die Erfindung betrifft ein Strahlendurchtrittsfenster sowie ein Herstellungsverfahren für ein Strahlendurchtrittsfenster.

Ein derartiges Strahlendurchtrittsfenster ist z.B. im Vakuumgehäuse einer Röntgenröhre und/oder in einem Strahlergehäuse eines Röntgenstrahlers eingebaut, in dem die Röntgenröhre angeordnet ist.

Sowohl das Vakuumgehäuse als auch das Strahlergehäuse muss eine entsprechende Stabilität aufweisen. Aus Gründen der Stabilität erfordert die Montage eines derartigen Strahlendurchtrittsfensters ein entsprechendes Bauvolumen der Röntgenröhre und/oder des Strahlergehäuses. Beispielsweise einen Tubus, der am Strahlergehäuse angeordnet ist (als Teil des Strahlergehäuses) und in dem das Strahlendurchtrittsfenster mechanisch stabil gehaltert ist.

Weiterhin sind derartige Strahlendurchtrittsfenster in Angio-MR-Systemen angeordnet. Ein derartiges Bildgebungssystem ist in der am 06.09.2017 eingereichten europäischen Patentanmeldung mit der Anmeldenummer 17189602.0 beschrieben. Das Bildgebungssystem umfasst ein Magnetresonanztomografie(MRT)-Gerät für eine MR-Bildgebung und eine Röntgenquelle. Das MRT-Gerät umfasst ein Vakuumgefäß (Vakuumrezipient), das einen Untersuchungsraum umgibt und in dem wenigstens eine Zylinderspule zur Erzeugung eines Magnetfeldes angeordnet ist. Die Röntgenquelle ist in einem Röntgenstrahler angeordnet, wobei der Röntgenstrahler in einer ersten Variante im MRT-Gerät und gemäß einer zweiten Variante außen am MRT-Gerät angeordnet ist. Falls der Röntgenstrahler bei diesem Bildgebungssystem an der Außenseite des MRT-Gerätes angeordnet ist, muss der Strahlengang durch den Vakuumrezipienten des MRT-Geräts verlaufen. Die Röntgenstrahlung tritt somit durch ein Strahleneintrittsfenster in das Vakuumgefäß ein und durch ein Strahlenaustrittsfenster aus dem Vakuumgefäß aus um anschließend in den Untersuchungsraum einzutreten.

Aufgabe der vorliegenden Erfindung ist es, ein Strahlendurchtrittsfenster zu schaffen, das auf einfache Weise auf den jeweiligen Anwendungsfall anpassbar ist.

Der Erfindung liegt weiterhin die Aufgabe zugrunde, hierfür ein geeignetes Herstellungsverfahren anzugeben.

Die Aufgabe wird erfindungsgemäß durch ein Strahlendurchtrittsfenster nach Anspruch 1 gelöst. Vorteilhafte Ausgestaltungen des erfindungsgemäßen Strahlendurchtrittsfensters sind jeweils Gegenstand der Ansprüche 2 bis 10. Hinsichtlich des Herstellungsverfahrens für ein Strahlendurchtrittsfenster wird die Aufgabe erfindungsgemäß durch ein Herstellungsverfahren nach Anspruch 11 gelöst, wobei vorteilhafte Ausgestaltungen in den Ansprüchen 12 und 13 beschrieben sind. Ein Bildgebungssystem mit zumindest einem erfindungsgemäßen Strahlenaustrittsfenster ist Gegenstand von Anspruch 14.

Das Strahlendurchtrittsfenster nach Anspruch 1 besteht aus einem Schichtverbund-Werkstoff und umfasst ein erstes Material und ein zweites Material. Das erste Material ist ein Trägermaterial, das einen Fensterrahmen bildet, und das zweite Material ist ein strahlendurchlässiges Material (Fenstermaterial) .

Das in Anspruch 11 definierte Herstellungsverfahren für ein Strahlendurchtrittsfenster umfasst
- einen ersten Fertigungsschritt, in dem durch ein Explosionsschweißverfahren ein Schichtverbund-Werkstoff aus einem ersten Material und aus einem zweiten Material hergestellt wird,
- einen zweiten Fertigungsschritt, in dem im Strahlendurchtrittsbereich das erste Material durch ein erstes Materialabtragungsverfahren entfernt wird.

Das Strahlendurchtrittsfenster nach Anspruch 1 bzw. das nach dem Herstellungsverfahren gemäß Anspruch 11 gefertigte Strahlendurchtrittsfenster ist für eine Vielzahl von Röntgenröhren und Strahlergehäusen geeignet. So kann das erfindungsgemäße Strahlendurchtrittsfenster beispielsweise in einer Röntgenröhre (Vakuumgehäuse) aus einem Metall (z.B. Stahl oder Aluminium) oder einer Metallkeramik eingebaut werden, wobei die erforderliche hohe Vakuumdichtheit durch die erfindungsgemäße Lösung gewährleistet ist. Auch für einen Einbau in einem Strahlergehäuse aus einem Metall (z.B. Aluminium oder Stahl) ist das erfindungsgemäße Strahlendurchtrittsfenster geeignet. Damit ergibt sich für das Vakuumgehäuse bzw. das Strahlergehäuse eine reduzierte Baugröße, da das Strahlendurchtrittsfenster an die erforderliche Einbaugeometrie anpassbar ist.

Durch den Einbau eines derartigen Strahlendurchtrittsfensters in eine Röntgenröhre bzw. in ein Strahlergehäuse erhält man im Bereich der Strahlendurchdringung eine ausreichende Stabilität bei gleichzeitig minimaler Strahlenschwächung.

Das Strahlendurchtrittsfenster nach Anspruch 1 ist für einen Einsatz in einem Bildgebungssystem gemäß Anspruch 14 geeignet, das ein MRT-Gerät und eine Röntgenquelle umfasst. Die Röntgenquelle ist z.B. in einer Röntgenröhre bzw. in einem Röntgenstrahler angeordnet.

Bei dem erfindungsgemäßen Bildgebungssystem weist
- das MRT-Gerät ein Vakuumgefäß (Vakuumrezipient) auf, das einen Untersuchungsraum umgibt und in dem wenigstens eine Zylinderspule zur Erzeugung eines Magnetfeldes angeordnet ist, wobei
- die Röntgenquelle derart am Außenumfang des MRT-Gerätes angeordnet ist, dass die Röntgenstrahlung durch ein Strahleneintrittsfenster in das Vakuumgefäß eintritt und durch ein Strahlenaustrittsfenster aus dem Vakuumgefäß austritt und in den Untersuchungsraum eintritt.

Erfindungsgemäß sind
- das Strahleneintrittsfenster als Strahlendurchtrittsfenster nach wenigstens einem der Ansprüche 1 bis 10 ausgebildet und/oder
- das Strahlenaustrittsfenster als Strahlendurchtrittsfenster nach wenigstens einem der Ansprüche 1 bis 10 ausgebildet.

Gemäß einer besonders vorteilhaften Ausführungsform des erfindungsgemäßen Strahlendurchtrittsfensters ist der Schichtverbund-Werkstoff durch ein Explosionsschweißverfahrenen hergestellt (Anspruch 2). Im Bereich der mikroskopisch kleinen Verbindungszone erfolgt eine lokale plastische Verformung, ohne dass eine Diffusion stattfindet. Dadurch können auch unterschiedliche Materialien, im vorliegenden Fall Trägermaterial und Fenstermaterial, dauerhaft miteinander verbunden werden.

Vorzugsweise ist das zweite Material (Fenstermaterial) für eine Durchlässigkeit von Röntgenstrahlen optimiert (Anspruch 3). Diese Optimierung ist durch eine Anpassung der Materialdicke und/oder der Strahlendurchlässigkeit erzielbar.

Im Hinblick auf den Aufbau des Strahlendurchtrittsfensters sind für das erste Material (Trägermaterial) und für das zweite Material (Fenstermaterial) die nachfolgend aufgeführten Materialien besonders vorteilhaft.

Gemäß einer bevorzugten Ausgestaltung (Anspruch 4) ist das erste Material, das das Trägermaterial bildet, eine Stahllegierung.

Die Stahllegierung ist in vorteilhafter Weise eine rostfreie und säurebeständige Stahllegierung (Anspruch 5).

Eine besonders bevorzugte Stahllegierung besitzt die Werkstoffnummer 1.4301 (Anspruch 6). Bei diesem hochlegierten Stahl mit dem Kurznamen X5CrNi18-10 handelt es sich um einen nichtrostenden austenitischen Chrom-Nickelstahl.

Für das zweite Material (Fenstermaterial) des Strahlendurchtrittsfensters ist vorzugsweise eine Aluminiumlegierung (Anspruch 7) oder eine Titanlegierung (Anspruch 8) vorzusehen.

Eine besonders geeignete Aluminiumlegierung ist AlMg₄ (Anspruch 9), die als Hauptlegierungselement Magnesium (Mg) enthält und auch als EN AW-5086 bezeichnet wird. Hierbei handelt es sich um eine Knetlegierung, die sich zur Bearbeitung durch Umformen (z.B. Schmieden, Walzen oder Biegen) eignet.

Eine bevorzugte Titanlegierung ist TiAl₆V₄ (Anspruch 10). Bei dieser Legierung handelt es sich um eine Alpha-Beta-TitanLegierung, die 90 Gew.-% Titan (Ti), 6 Gew.-% Aluminium (Al) und 4 % Vanadium (V) enthält. Diese Legierung weist eine hohe Festigkeit bei geringem Gewicht sowie eine sehr gute Korrosionsbeständigkeit auf.

Sowohl die Aluminiumlegierung nach Anspruch 9 als auch die Titanlegierung nach Anspruch 10 erlauben eine entsprechend geringe Schichtdicke des zweiten Materials (strahlendurchlässiges Fenstermaterial), so dass das Strahlendurchtrittsfenster entsprechend dünn ausgelegt werden kann, wodurch die Strahlschwächung entsprechend minimiert wird.

Durch das homogene Gefüge der verwendeten Aluminiumlegierung und durch das homogene Gefüge der verwendeten Titanlegierung sowie durch die einheitliche Dicke des Strahlendurchtrittsfensters im Bereich der Röntgenstrahlung werden Bildartefakte zuverlässig verhindert.

Bei speziellen Einbauverhältnissen, die z.B. bei einem Magnetresonanztomografie(MRT)-Gerät auftreten können, kann es vorteilhaft sein, das Herstellverfahren gemäß Anspruch 11 um wenigstens einen der folgenden Fertigungsschritte zu ergänzen:
- Einen dritten Fertigungsschritt, in dem das zweite Material durch ein zweites Materialabtragungsverfahren auf eine einheitliche Schichtdicke gebracht wird (Anspruch 12),
- einen vierten Fertigungsschritt, in dem das Strahlendurchtrittsfenster in einem Kaltumformverfahren an die erforderliche Einbaugeometrie angepasst wird (Anspruch 13).

Nachfolgend wird ein schematisch dargestelltes Ausführungsbeispiel der Erfindung anhand der Zeichnung näher erläutert, ohne jedoch darauf beschränkt zu sein. Es zeigen:
- FIG 1: eine perspektivische Darstellung eines Strahlendurchtrittsfensters,
- FIG 2: das Strahlendurchtrittsfenster gemäß FIG 1 im eingebauten Zustand,
- FIG 3: einen Teil-Längsschnitt eines Bildgebungssystems mit einem MRT-Gerät und einer Röntgenquelle.

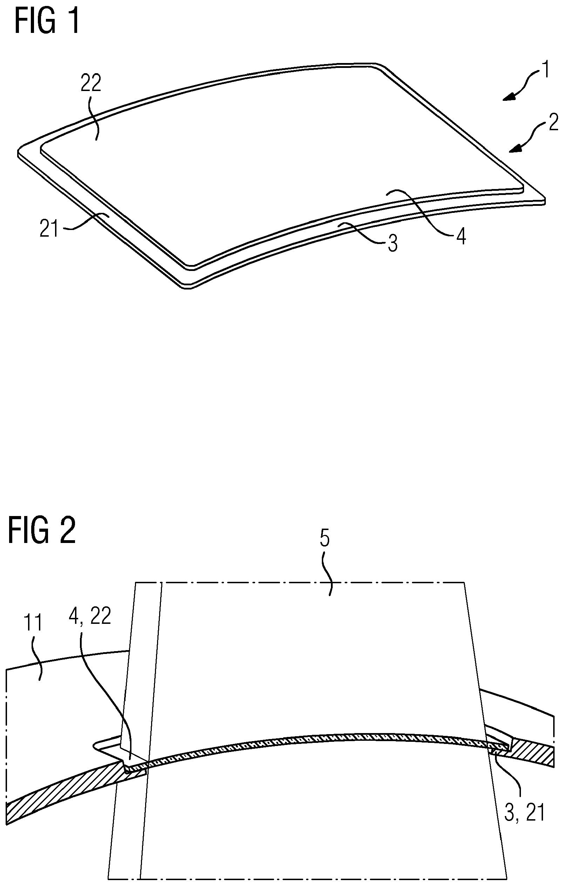

In FIG 1 ist mit 1 Strahlendurchtrittsfenster bezeichnet, das erfindungsgemäß aus einem Schichtverbund-Werkstoff 2 besteht. Der Schichtverbund-Werkstoff 2 umfasst ein erstes Material 21 und ein zweites Material 22. Das erste Material 21 ist ein Trägermaterial und einen bildet einen Fensterrahmen 3. Das zweite Material 22 ist ein strahlendurchlässiges Material und bildet somit das eigentliche strahlendurchlässige Fenster 4.

Ein Verfahren zur Herstellung des in FIG 1 dargestellten Strahlendurchtrittsfensters 1 umfasst folgende erfindungsgemäße Fertigungsschritte:
- In einem ersten Fertigungsschritt wird durch ein Explosionsschweißverfahrenen ein Schichtverbund-Werkstoff 2 aus einem ersten Material 21 und aus einem zweiten Material 22 hergestellt (Sandwich-Konstruktion),
- in einem zweiten Fertigungsschritt wird im Strahlendurchtrittsbereich das erste Material 21 (Trägermaterial) durch ein erstes Materialabtragungsverfahren (z.B. spanende Abtragung) entfernt.

Da das Strahlendurchtrittsfenster 1 in einem Vakuumrezipienten 11 eines Magnetresonanztomografie(MRT)-Gerätes 10 eingebaut werden soll, umfasst das Herstellungsverfahren noch folgende weitere Maßnahmen:
- In einem dritten Fertigungsschritt wird das zweite Material 22 (Fenstermaterial) durch ein zweites Materialabtragungsverfahren (z.B. Fräsen) auf eine einheitliche Schichtdicke gebracht,
- in einem vierten Fertigungsschritt wird das Strahlendurchtrittsfenster 1 in einem Kaltumformverfahren an die erforderliche Einbaugeometrie angepasst.

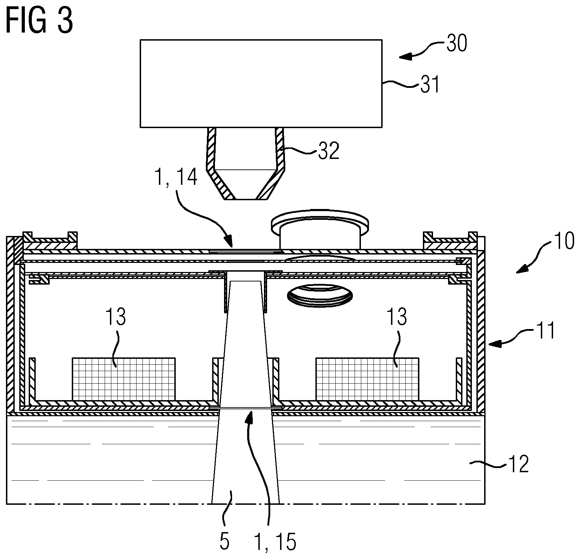

Nach dem Anpassen des Strahlendurchtrittsfensters 1 an die erforderliche Einbaugeometrie wird das Strahlendurchtrittsfenster 1 über den Fensterrahmen 3, der aus dem Trägermaterial 21 besteht, in einen Vakuumrezipienten 11 eines Angio-MR-Geräts, z.B. einem Bildgebungssystem gemäß FIG 3, eingeschweißt. Das Einschweißen kann beispielsweise mittels Wolfram-Inertgas-Schweißen erfolgen. Man erhält dadurch im Bereich der Strahlendurchdringung eine ausreichende Stabilität bei gleichzeitig minimaler Strahlenschwächung der Röntgenstrahlung 5 beim Durchtritt durch das Fenster 4.

In FIG 2 ist das Strahlendurchtrittsfenster 1 im eingebauten Zustand dargestellt. Der Fensterrahmen 3 (bestehend aus dem ersten Material 21) ist stoffschlüssig im Vakuumrezipienten 11 eingebaut. Die durch das strahlendurchlässige Fenster 4 (bestehend aus dem zweiten Material 22) durchtretende Röntgenstrahlung 5 bildet einen Strahlenkegel.

Durch das Herstellungsverfahren des Rohlings für das Strahlendurchtrittsfenster können Materialien stoffschlüssig miteinander verbunden werden, ohne zusätzlich Bauraum für Verbindungsgeometrien, wie z.B. Lötgeometrien oder Schweißlippen, zu benötigen. Dadurch können die minimalen Bauraumanforderungen im Angio-MR-Gerät, insbesondere im Vakuumrezipienten erfüllt werden. Es steht eine größere Auswahl an Werkstoffen zur Auswahl, um das Fenster zu realisieren.

Durch Auswahl einer passenden Aluminium- oder Titanlegierung kann das strahlendurchlässige Fenster 4 entsprechend dünn ausgelegt werden und die Schwächung der Röntgenstrahlung 5 entsprechend minimiert werden. Eine bevorzugte Aluminiumlegierung für das Fenstermaterial 22 (zweites Material) ist AlMg₄. Eine besonders geeignete Titanlegierung für das Fenstermaterial 22 ist TiAl₆V₄.

Das homogene Gefüge der verwendeten Aluminium- und Titanlegierungen und die einheitliche Dicke des strahlendurchlässigen Fensters 2 im Bereich des Röntgenstrahls 5 verhindern Bildartefakte.

Das in FIG 3 in einem Teil-Längsschnitt dargestellte Bildgebungssystem umfasst ein Magnetresonanztomografie(MRT)-Gerät 10 und eine Röntgenquelle 30.

Die Röntgenquelle 30 umfasst eine nicht näher dargestellte Röntgenröhre, die in einem Strahlergehäuse 31 mit einer Blende 32 angeordnet ist. Die Röntgenquelle 30 ist somit als Röntgenstrahler ausgebildet.

Das MRT-Gerät 10 weist ein Vakuumgefäß auf, das als Vakuumrezipient 11 ausgebildet ist. Der Vakuumrezipient 11 umgibt einen kreiszylindrisch ausgebildeten Untersuchungsraum 12, der zur Aufnahme eines nicht dargestellten Patienten dient. Im Vakuumrezipienten 11 sind im dargestellten Ausführungsbeispiel zwei Zylinderspulen 13 zur Erzeugung eines Magnetfeldes angeordnet.

Der Röntgenstrahler 30 ist derart am Außenumfang des MRT-Gerätes 10 angeordnet, dass die Röntgenstrahlung 5, die einen Strahlenkegel bildet, durch ein Strahleneintrittsfenster 14 in den 11 eintritt und durch ein Strahlenaustrittsfenster 15 aus dem Vakuumrezipienten 11 austritt und in den Untersuchungsraum 12 eintritt.

Sowohl das Strahleneintrittsfenster 14 als auch das Strahlenaustrittsfenster 15 ist erfindungsgemäß als Strahlendurchtrittsfenster 1 nach FIG 1 ausgeführt, wobei der Einbau in den Vakuumrezipienten 11 beispielsweise entsprechend FIG 2 vorgenommen wurde.

## Patentansprüche

1. Strahlendurchtrittsfenster, das aus einem Schichtverbund-Werkstoff (2) besteht, der ein erstes Material (21) und ein zweites Material (22) umfasst, wobei das erste Material (21) ein Trägermaterial ist, das einen Fensterrahmen (3) bildet, und das zweite Material (22) ein strahlendurchlässiges Material ist.

2. Strahlendurchtrittsfenster nach Anspruch 1, **dadurch gekennzeichnet, dass** der Schichtverbund-Werkstoff (2) durch ein Explosionsschweißverfahrenen hergestellt ist.

3. Strahlendurchtrittsfenster nach Anspruch 1, **dadurch gekennzeichnet, dass** das zweite Material (22) für eine Durchlässigkeit von Röntgenstrahlen optimiert ist.

4. Strahlendurchtrittsfenster nach Anspruch 1, **dadurch gekennzeichnet, dass** das erste Material (21) eine Stahllegierung ist.

5. Strahlendurchtrittsfenster nach Anspruch 4, **dadurch gekennzeichnet, dass** die Stahllegierung eine rostfreie und säurebeständige Stahllegierung ist.

6. Strahlendurchtrittsfenster nach Anspruch 4, **dadurch gekennzeichnet, dass** die Stahllegierung die Werkstoffnummer 1.4301 besitzt

7. Strahlendurchtrittsfenster nach Anspruch 1, **dadurch gekennzeichnet, dass** das zweite Material (22) eine Aluminiumlegierung ist.

8. Strahlendurchtrittsfenster nach Anspruch 1, **dadurch gekennzeichnet, dass** das zweite Material (22) eine Titanlegierung ist.

9. Strahlendurchtrittsfenster nach Anspruch 7, **dadurch gekennzeichnet, dass** die Aluminiumlegierung AlMg₄ ist.

10. Strahlendurchtrittsfenster nach Anspruch 8, **dadurch gekennzeichnet, dass** die Titanlegierung TiAl₆V₄ ist.

11. Herstellungsverfahren für ein Strahlendurchtrittsfenster (1), bei dem
- in einem ersten Fertigungsschritt durch ein Explosionsschweißverfahrenen ein Schichtverbund-Werkstoff (2) aus einem ersten Material (21) und aus einem zweiten Material (2) hergestellt wird,
- in einem zweiten Fertigungsschritt im Strahlendurchtrittsbereich das erste Material (21) durch ein erstes Materialabtragungsverfahren entfernt wird.

12. Herstellungsverfahren nach Anspruch 11, bei dem
- in einem dritten Fertigungsschritt das zweite Material (22) durch ein zweites Materialabtragungsverfahren auf eine einheitliche Schichtdicke gebracht wird,

13. Herstellungsverfahren nach Anspruch 11 oder 12, bei dem
- in einem vierten Fertigungsschritt das Strahlendurchtrittsfenster (1) in einem Kaltumformverfahren an die erforderliche Einbaugeometrie angepasst wird.

14. Bildgebungssystem mit einem MRT-Gerät (10) und einer Röntgenquelle (30),
- wobei das MRT-Gerät (10) ein Vakuumgefäß (11) umfasst, das einen Untersuchungsraum (12) umgibt und in dem wenigstens eine Zylinderspule (13) zur Erzeugung eines Magnetfeldes angeordnet ist,
- und wobei die Röntgenquelle (30) derart am Außenumfang des MRT-Gerätes (10) angeordnet ist, dass die Röntgenstrahlung durch ein Strahleneintrittsfenster (14) in das Vakuumgefäß (11) eintritt und durch ein Strahlenaustrittsfenster (15) aus dem Vakuumgefäß (11) austritt und in den Untersuchungsraum (12) eintritt,
**dadurch gekennzeichnet, dass**
- das Strahleneintrittsfenster (14) ein Strahlendurchtrittsfenster (1) nach einem der Ansprüche 1 bis 10 ist und/oder
- das Strahlenaustrittsfenster (15) ein Strahlendurchtrittsfenster (1) nach einem der Ansprüche 1 bis 10 ist.
